# EUROPEAN PATENT SPECIFICATION

(11) **EP 1 496 909 B1**
(45) Date of publication and mention of the grant of the patent: **14.02.2007**
(21) Application number: 03712464.1
(22) Date of filing: 14.04.2003
(51) Int. Cl.: A61K 31/517, A61K 31/165, A61P 35/00

(54) **COMBINATION THERAPY FOR THE TREATMENT OF CANCER**
KOMBINATIONSTHERAPIE ZUR BEHANDLUNG VON KREBS
THERAPIE COMBINEE DE TRAITEMENT DU CANCER

(30) Priority: 16.04.2002 GB 0208680; 08.08.2002 GB 0218388
(43) Date of publication of application: 19.01.2005
(73) Proprietor: AstraZeneca AB, S-151 85 Södertälje (SE)
(72) Inventor: RYAN, Anderson, Joseph AstraZeneca R & D Alderley, Macclesfield Cheshire SK10 4TG (GB)
(86) International application number: PCT/GB2003/001617
(87) International publication number: WO 2003/088971

(56) References cited:
- WO-A-96/33980
- WO-A-99/02166
- CIARDIELLO F ET AL: "A novel approach in the treatment of cancer: targeting the epidermal growth factor receptor." CLINICAL CANCER RESEARCH: AN OFFICIAL JOURNAL OF THE AMERICAN ASSOCIATION FOR CANCER RESEARCH. UNITED STATES OCT 2001, vol. 7, no. 10, October 2001 (2001-10), pages 2958-2970, XP002248479 ISSN: 1078-0432

## Description

The present invention relates to a pharmaceutical composition comprising ZD6126 and ZD1839; to a kit comprising ZD6126 and ZD1839; and to the use of ZD6126 and ZD1839 in the manufacture of a medicament suitable for the production of a vascular damaging effect resulting from inappropriate angiogenesis in a warm-blooded animal such as a human which is optionally being treated with ionising radiation, and in particular suitable for the treatment of a cancer involving a solid tumour.

Normal angiogenesis plays an important role in a variety of processes including embryonic development, wound healing and several components of female reproductive function. Undesirable or pathological angiogenesis has been associated with disease states including diabetic retinopathy, psoriasis, cancer, rheumatoid arthritis, atheroma, Kaposi's sarcoma and haemangioma (Fan et al, 1995, Trends Pharmacol. Sci. 16: 57-66; Folkman, 1995, Nature Medicine 1: 27-31). Formation of new vasculature by angiogenesis is a key pathological feature of several diseases (J. Folkman, New England Journal of Medicine 333, 1757-1763 (1995)). For example, for a solid tumour to grow it must develop its own blood supply upon which it depends critically for the provision of oxygen and nutrients; if this blood supply is mechanically shut off the tumour undergoes necrotic death. Neovascularisation is also a clinical feature of skin lesions in psoriasis, of the invasive pannus in the joints of rheumatoid arthritis patients and of atherosclerotic plaques. Retinal neovascularisation is pathological in macular degeneration and in diabetic retinopathy.

Reversal of neovascularisation by damaging the newly-formed vascular endothelium is expected to have a beneficial therapeutic effect. International Patent Application Publication No. WO 99/02166 describes tricyclic compounds that surprisingly have a selective damaging effect on newly formed vasculature as compared to the normal, established vascular endothelium of the host species. This is a property of value in the treatment of disease states associated with angiogenesis such as cancer, diabetes, psoriasis, rheumatoid arthritis, Kaposi's sarcoma, haemangioma, lymphoedema, acute and chronic nephropathies, atheroma, arterial restenosis, autoimmune diseases, acute inflammation, excessive scar formation and adhesions, endometriosis, dysfunctional uterine bleeding and ocular diseases with retinal vessel proliferation including macular degeneration.

Compounds which damage newly formed vasculature are vascular targeting agents (VTAs) and are also known as vascular damaging agents (VDAs).

One such compound described in International Patent Application Publication No. WO 99/02166 is N-acetylcolchiriol-O-phosphate, (also know as (5*S*)-5-(acetylaunino)-9,10,11-trimethoxy-6,7-dihydro-5*H*-dibenzo[a,cJcyclohepten-3-yl dihydrogen phosphate; Example 1 of WO 99/02166), which is referred to herein as ZD6126:

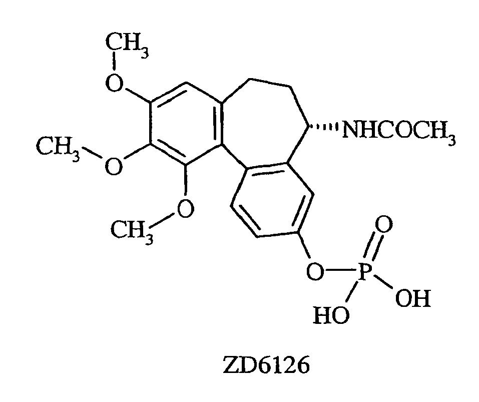

It is believed that ZD6126 damages newly-formed vasculature, for example the vasculature of tumours, thus effectively reversing the process of angiogenesis. It has been reported that ZD6126 selectively disrupts tumour vasculature leading to vessel occlusion and extensive tumour necrosis (Davis PD, Hill SA, Galbraith SM, et al. Proc. Am. Assoc. Cancer Res. 2000; 41: 329).

In WO 99/02166 it is stated that:
"compounds of the invention may be administered as sole therapy or in combination with other treatments. For the treatment of solid tumours compounds of the invention may be administered in combination with radiotherapy or in combination with other anti-tumour substances for example those selected from mitotic inhibitors, for example vinblastine, paclitaxel and docetaxel; alkylating agents, for example cisplatin, carboplatin and cyclophosphamide, antimetabolites, for example 5-fluorouracil, cytosine arabinoside and hydroxyurea; intercalating agents for example adriamycin and bleomycin; enzymes, for example asparaginase; topoisomerase inhibitors for example etoposide, topotecan and irinotecan; thymidylate synthase inhibitors for example raltitrexed; biological response modifers for example interferon; antibodies for example edrecolomab, and anti-hormones for example tamoxifen. Such combination treatment may involve simultaneous or sequential application of the individual components of the treatment."

Nowhere in WO 99/02166 does it suggest any combination of a VTA and an epidermal growth factor receptor tyrosine kinase inhibitor for the treatment of any disease state including cancer.

Nowhere in WO 99/02166 is the specific combination of ZD6126 and ZD1839 suggested.

Nowhere in WO 99/02166 does it state that use of any compound of the invention therein with other treatments will produce surprisingly beneficial effects.

ZD 1839 is *N*-(3-chloro-4-fluorophenyl)-7-methoxy-6-(3-morpholinopropoxy)quinazolin-4-amine:

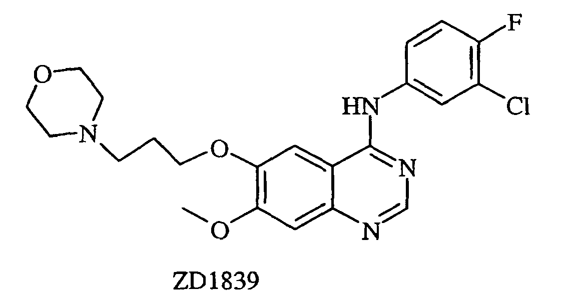

ZD1839 is also known as Iressa^{™} (Trademark of AstraZeneca UK Limited) and it is an epidermal growth factor receptor (EGFR) tyrosine kinase inhibitor (TKI). ZD1839 is described in International Patent Application Publication No. WO 96/33980.

In recent years it has been discovered that certain growth factor tyrosine kinase enzymes are important in the transmission of biochemical signals which initiate cell replication. They are large proteins which span the cell membrane and possess an extracellular binding domain for growth factors, for example the epidermal growth factor receptor (EGFR) which binds epidermal growth factor (EGF), and an intracellular portion which functions as a kinase to phosphorylate tyrosine amino acids in proteins and hence to influence cell proliferation.

EGFR is a member of the erbB family of receptor tyrosine kinases, which includes EGFR, erbB2, erbB3 and erbB4, and it is known that these receptor tyrosine kinases are frequently involved in driving the proliferation and survival of tumour cells (reviewed in Olayioye et al., EMBO J., 2000, 19, 3159). One mechanism by which this can be accomplished is by overexpression of the receptor at the protein level, generally as a result of gene amplification. This has been observed in many common human cancers (reviewed in Klapper et al., Adv. Cancer Res., 2000, 77, 25), such as breast cancer (Sainsbury et al., Brit. J. Cancer, 1988, 58, 458; Guerin et al., Oncogene Res., 1988, 3, 21; Slamon et al., Science, 1989, 244, 707; Klijn et al., Breast Cancer Res. Treat., 1994, 29, 73 and reviewed in Salomon et al., Crit. Rev. Oncol. Hematol., 1995, 19, 183), non-small cell lung cancers (NSCLCs) including adenocarcinomas (Cerny et al., Brit. J. Cancer, 1986, 54, 265; Reubi et al., Int. J. Cancer, 1990, 45, 269; Rusch et al., Cancer Research, 1993, 53, 2379; Brabender et al, Clin. Cancer Res., 2001, 7, 1850) as well as other cancers of the lung (Hendler et al., Cancer Cells, 1989, 7, 347; Ohsaki et al., Oncol. Rep., 2000, 7, 603), bladder cancer (Neal et al., Lancet, 1985, 366; Chow et al., Clin. Cancer Res., 2001, 7, 1957, Zhau et al., Mol Carcinog., 3, 254), oesophageal cancer (Mukaida et al., Cancer, 1991, 68, 142), gastrointestinal cancer such as colon, rectal or stomach cancer (Bolen et al., Oncogene Res., 1987, 1, 149; Kapitanovic et al., Gastroenterology, 2000, 112, 1103; Ross et al., Cancer Invest., 2001, 19, 554), cancer of the prostate (Visakorpi et al., Histochem J., 1992, 24, 481; Kumar et al., 2000, 32, 73; Scher et al., J. Natl. Cancer Inst., 2000, 92, 1866), leukaemia (Konaka et al., Cell, 1984, 37, 1035, Martin-Subero et al., Cancer Genet Cytogenet., 2001, 127, 174), ovarian cancer (Hellstrom et al., Cancer Res., 2001, 61, 2420), head and neck cancer (Shiga *et al*., Head Neck, 2000, 22, 599) and pancreatic cancer (Ovotny et al, Neoplasma, 2001, 48, 188).

It is widely believed that as a consequence of the dysfunctional regulation of one or more of these receptors many tumours become clinically more aggressive and this correlates with a poorer prognosis for the patient (Brabender et al, Clin. Cancer Res., 2001, 7, 1850; Ross et al, Cancer Investigation, 2001, 19, 554, Yu et al., Bioessays, 2000, 22.7, 673). In addition to these clinical findings, a wealth of pre-clinical information suggests that the erbB family of receptor tyrosine kinases are involved in cellular transformation. This includes the observations that many tumour cell lines overexpress one or more of the erbB receptors and that EGFR or erbB2 when transfected into non-tumour cells have the ability to transform these cells. In addition to this, a number of pre-clinical studies have demonstrated that anti-proliferative effects can be induced by knocking out one or more erbB activities by small molecule inhibitors, dominant negatives or inhibitory antibodies (reviewed in Mendelsohn et al., Oncogene, 2000, 19, 6550). Thus it has been recognised that inhibitors of these receptor tyrosine kinases should be of value as selective inhibitors of the proliferation of mammalian cancer cells (Yaish *et al.* Science, 1988, 242, 933, Kolibaba *et. al*, Biochimica et Biophysica Acta, 1997, 133, F217-F248; Al-Obeidi *et al*, 2000, Oncogene, 19, 5690-5701; Mendelsohn *et al*, 2000, Oncogene, 19, 6550-6565). In addition to this pre-clinical data, the use of inhibitory antibodies against EGFR and erbB2 (c-225 and trastuzumab respectively) has proven to be beneficial in the clinic for the treatment of selected solid tumours (reviewed in Mendelsohn *et al,* 2000, Oncogene, 19, 6550-6565).

It is believed that members of the erbB type receptor tyrosine kinase family may be implicated in a number of non-malignant proliferative disorders because amplification and/or activity of erbB receptor tyrosine kinases has been detected in psoriasis (Ben-Bassat, Curr. Pharm. Des., 2000, 6, 933; Elder et al., Science, 1989, 243, 811), benign prostatic hyperplasia (BPH) (Kumar et al., Int. Urol. Nephrol., 2000, 32,73), atherosclerosis and restenosis (Bokemeyer et al., Kidney Int., 2000, 58, 549). It is therefore expected that inhibitors of erbB type receptor tyrosine kinases will be useful in the treatment of these and other non-malignant disorders involving excessive cellular proliferation.

It is known from International Patent Application Publication No. WO 96/33980 that ZD1839 possesses EGFR tyrosine kinase inhibitory activity (J R Woodburn *et al*. in Proc. Amer. Assoc. Cancer Research, 1997, 38, 633 and Pharmacol. Ther., 1999, 82, 241-250; also A.E. Wakeling *et al*. in Cancer Research, 2002, 62, 5749-5754) and is an inhibitor of the proliferation of cancer tissue.

It is stated in WO 96/33980 that compounds of the invention, which include ZD1839, may be given conjointly with other cancer therapies. It states therein "The anti-proliferative treatment defined hereinbefore may be applied as a sole therapy or may involve, in addition to the quinazoline derivative of the invention, one or more other anti-tumour substances, for example cytotoxic or cytostatic anti-tumour substances, for example those selected from, for example, mitotic inhibitors, for example vinblastine, vindesine and vinorelbine; tubulin disassembly inhibitors such as taxol; alkylating agents, for example cis-platin, carboplatin and cyclophosphamide; antimetabolites, for example 5-fluorouracil, tegafur, methotrexate, cytosine arabinoside and hydroxyurea, or, for example, one of the preferred antimetabolites disclosed in European Patent Application No. 239362 such as N-{5-[N-(3,4-dihydro-2-methyl-4-oxoquinazolin-6-ylmethyl)-N-methylamino]-2-thenoyl}-L-glutamic acid; intercalating antibiotics, for example adriamycin, mitomycin and bleomycin; enzymes, for example asparaginase; topoisomerase inhibitors, for example etoposide and camptothecin; biological response modifiers, for example interferon; anti-honnones, for example antioesti-ogens such as tamoxifen, for example antiandrogens such as 4'-cyano-3-(4-fluorophenylsulphonyl)-2-hydroxy-2-methyl-3'-(trifluoromethyl)-propionanilide or, for example LHRH antagonists or LHRH agonists such as goserelin, leuprorelin or buserelin and hormone synthesis inhibitors, for example aromatase inhibitors such as those disclosed in European Patent Application No. 0296749, for example 2,2'-[5-(1H-1,2,4- triazol-1-ylmethyl)-1,3-phenylene]bis(2-methylpropionitrile), and, for example, inhibitors of 5α-reductase such as 17β-(N-tert-butylcarbamoyl)-4-aza-5α-androst-1-en-3-one."

Nowhere in WO 96/33980 does it suggest any combination of a VTA and an EGFR TKI for the treatment of any disease state including cancer. Vascular damaging effects and angiogenesis are not discussed in WO 96/33980.

Unexpectedly and surprisingly we have now found that the particular compound ZD6126 used in combination with ZD1839, produces significantly better anti-tumour effects than each of ZD6126 and ZD 1839 used alone.

Anti-cancer effects of the pharmaceutical composition of the present invention include, anti-tumour effects, the response rate, the time to disease progression and the survival rate. Anti-tumour effects of the pharmaceutical composition of the present invention include inhibition of tumour growth, tumour growth delay, regression of tumour, shrinkage of tumour, increased time to regrowth of tumour on cessation of treatment, slowing of disease progression. It is expected that when the pharmaceutical composition of the present invention is administered to a warm-blooded animal such as a human, in need of treatment for cancer involving a solid tumour, said composition will produce an effect, as measured by, for example, one or more of: the extent of the anti-tumour effect, the response rate, the time to disease progression and the survival rate.

According to the present invention there is provided a composition which produces a vascular damaging effect in a warm-blooded animal such as a human, which comprises an effective amount of ZD6126 or a pharmaceutically acceptable salt thereof which is administered to said animal before, after or simultaneously with an effective amount of ZD1839 or a pharmaceutically acceptable salt thereof.

According to a further aspect of the present invention there is provided a composition for the treatment of a cancer involving a solid tumour in a warm-blooded animal such as a human, which comprises an effective amount of ZD6126 or a pharmaceutically acceptable salt thereof which is administered to said animal before, after or simultaneously with an effective amount of ZD1839 or a pharmaceutically acceptable salt thereof.

According to a further aspect of the present invention there is provided a composition for the production of a vascular damaging effect resulting from inappropriate angiogenesis in a warm-blooded such as a human, which comprises an effective amount of ZD6126 or a pharmaceutically acceptable salt thereof which is administered to said animal before, after or simultaneously with an effective amount of ZD1839 or a pharmaceutically acceptable salt thereof, wherein ZD6126 and ZD 1839 may each optionally be administered together with a pharmaceutically acceptable excipient or carrier.

According to a further aspect of the present invention there is provided a composition for the treatment of a cancer involving a solid tumour in a warm-blooded animal such as a human, which comprises an effective amount of ZD6126 or a pharmaceutically acceptable salt thereof which is administered to said animal before, after or simultaneously with an effective amount of ZD1839 or a pharmaceutically acceptable salt thereof, wherein ZD6126 and ZD1839 may each optionally be administered together with a pharmaceutically acceptable excipient or carrier.

According to a further aspect of the invention there is provided a pharmaceutical composition which comprises ZD6126 or a pharmaceutically acceptable salt thereof, and ZD1839 or a pharmaceutically acceptable salt thereof, in association with a pharmaceutically acceptable excipient or carrier.

According to a further aspect of the present invention there is provided a combination product comprising ZD6126 or a pharmaceutically acceptable salt thereof and ZD1839 or a pharmaceutically acceptable salt thereof, for use in the treatment of a human or animal body by therapy.

According to a further aspect of the present invention there is provided a kit comprising ZD6126 or a pharmaceutically acceptable salt thereof, and ZD1839 or a pharmaceutically acceptable salt thereof.

According to a further aspect of the present invention there is provided a kit comprising:
a) ZD6126 or a pharmaceutically acceptable salt thereof in a first unit dosage form;
b) ZD1839 or a pharmaceutically acceptable salt thereof in a second unit dosage form; and
c) container means for containing said first and second dosage forms.

According to a further aspect of the present invention there is provided a kit comprising:
a) ZD6126 or a pharmaceutically acceptable salt thereof, together with a pharmaceutically acceptable excipient or carrier, in a first unit dosage form;
b) ZD1839 or a pharmaceutically acceptable salt thereof, together with a pharmaceutically acceptable excipient or carrier, in a second unit dosage form; and
c) container means for containing said first and second dosage forms.

According to a further aspect of the present invention there is provided the use of ZD6126 or a pharmaceutically acceptable salt thereof and ZD1839 or a pharmaceutically acceptable salt thereof in the manufacture of a medicament for use in the production of a vascular damaging effect resulting from inappropriate angiogenesis in a warm-blooded animal such as a human.

According to a further aspect of the present invention there is provided the use of ZD6126 or a pharmaceutically acceptable salt thereof and ZD1839 or a pharmaceutically acceptable salt thereof in the manufacture of a medicament for use in the production of an anti-cancer effect in a warm-blooded animal such as a human.

According to a further aspect of the present invention there is provided the use of ZD6126 or a pharmaceutically acceptable salt thereof and ZD1839 or a pharmaceutically acceptable salt thereof in the manufacture of a medicament for use in the production of an anti-tumour effect in a warm-blooded animal such as a human.

According to a further aspect of the present invention there is provided a therapeutic combination comprising an effective amount of ZD6126 or a pharmaceutically acceptable salt thereof, optionally together with a pharmaceutically acceptable excipient or carrier, and an effective amount of ZD1839 or a pharmaceutically acceptable salt thereof, optionally together with a pharmaceutically acceptable excipient or carrier, for simultaneous, sequential or separate administration to a warm-blooded animal such as a human in need of such therapeutic treatment. Such therapeutic treatment includes a vascular damaging effect resulting from inappropriate angiogenesis, an anti-cancer effect and an anti-tumour effect.

A combination treatment as defined herein may be achieved by way of the simultaneous, sequential or separate administration of the individual components of said treatment. A combination treatment as defined herein may be applied as a sole therapy or may involve surgery or radiotherapy.

Surgery may comprise the step of partial or complete tumour resection, prior to, during or after the administration of the combination treatment with ZD6126 described herein.

Unexpectedly and surprisingly we have found that the administration of a triple combination of ZD6126, ZD 1839 and ionising radiation produces anti-tumour effects greater than those achieved with any of the three therapies used alone, greater than those achieved with the combination of ZD6126 and ZD1839, greater than those achieved with the combination of ZD6126 and ionising radiation and greater than those achieved with the combination of ZD1839 and ionising radiation.

According to the present invention there is provided a pharmaceutical composition for the production of a vascular damaging effect resulting from inappropriate angiogenesis in a warm-blooded animal such as a human, which comprises an effective amount of ZD6126 or a pharmaceutically acceptable salt thereof which is administered to said animal before, after or simultaneously with an effective amount of ZD1839 or a pharmaceutically acceptable salt thereof and before, after or simultaneously with an effective amount of ionising radiation.

According to a further aspect of the present invention there is provided composition for the treatment of a cancer involving a solid tumour in a warm-blooded animal such as a human, which comprises an effective amount of ZD6126 or a pharmaceutically acceptable salt thereof which is administered to said animal before, after or simultaneously with an effective amount of ZD1839 or a pharmaceutically acceptable salt thereof and before, after or simultaneously with an effective amount of ionising radiation.

According to a further aspect of the present invention there is provided a composition for the production of a vascular damaging effect resulting from inappropriate angiogenesis in a warm-blooded animal such as a human, which comprises effective amount of ZD6126 or a pharmaceutically acceptable salt thereof which is administered to said animal before, after or simultaneously with an effective amount of ZD1839 or a pharmaceutically acceptable salt thereof and before, after or simultaneously with an effective amount of ionising radiation, wherein ZD6126 and ZD1839 may each optionally be administered together with a pharmaceutically acceptable excipient or carrier.

According to a further aspect of the present invention there is provided a composition for the treatment of a cancer involving a solid tumour in a warm-blooded animal such as a human, which comprises an effective amount of ZD6126 or a pharmaceutically acceptable salt thereof which is administered to said animal before, after or simultaneously with an effective amount of ZD 1839 or a pharmaceutically acceptable salt thereof and before, after or simultaneously with an effective amount of ionising radiation, wherein ZD6126 and ZD1839 may each optionally be administered together with a pharmaceutically acceptable excipient or carrier.

According to a further aspect of the present invention there is provided the use of ZD6126 or a pharmaceutically acceptable salt thereof and ZD1839 or a pharmaceutically acceptable salt thereof in the manufacture of a medicament for use in the production of a vascular damaging effect resulting from inappropriate angiogenesis in a warm-blooded animal such as a human which is being treated with ionising radiation.

According to a further aspect of the present invention there is provided the use of ZD6126 or a pharmaceutically acceptable salt thereof and ZD1839 or a pharmaceutically acceptable salt thereof in the manufacture of a medicament for use in the production of an anti-cancer effect in a warm-blooded animal such as a human which is being treated with ionising radiation.

According to a further aspect of the present invention there is provided the use of ZD6126 or a pharmaceutically acceptable salt thereof and ZD1839 or a pharmaceutically acceptable salt thereof in the manufacture of a medicament for use in the production of an anti-tumour effect in a warm-blooded animal such as a human which is being treated with ionising radiation.

According to a further aspect of the present invention there is provided a therapeutic combination comprising an effective amount of ZD6126 or a pharmaceutically acceptable salt thereof, optionally together with a pharmaceutically acceptable excipient or carrier, for simultaneous, sequential or separate administration with an effective amount of ZD1839 or a pharmaceutically acceptable salt thereof, optionally together with a pharmaceutically acceptable excipient or carrier which is administered before, after or simultaneously with an effective amount of ionising radiation, to a warm-blooded animal such as a human in need of such therapeutic treatment.

A warm-blooded animal such as a human which is being treated with ionising radiation means a warm-blooded animal such as a human which is treated with ionising radiation before, after or at the same time as the administration of a medicament or combination treatment comprising ZD6126 and ZD 1839. For example said ionising radiation may be given to said warm-blooded animal such as a human within the period of a week before to a week after the administration of a medicament or combination treatment comprising ZD6126 and ZD1839. This means that ZD6126, ZD 1839 and ionising radiation may be administered separately or sequentially in any order, or may be administered simultaneously. The warm-blooded animal may experience the effect of each of ZD6126, ZD1839 and radiation simultaneously.

According to one aspect of the present invention the ionising radiation is administered before one of ZD6126 and ZD1839 or after one of ZD6126 and ZD1839.

According to another aspect of the present invention the ionising radiation is administered before both ZD6126 and ZD1839 or after both ZD6126 and ZD1839.

In another aspect of the present invention ZD1839 is dosed daily continuously for a longer period of time during which time ZD6126 and ionising radiation are each administered periodically, that is for a few days, for example 1-5 days at a time.

According to another aspect of the present invention the effect of the composition of the present invention is expected to be at least equivalent to the addition of the effects of each of the components of said treatment used alone, that is, of each of ZD6126 and ZD1839 and ionising radiation used alone.

According to another aspect of the present invention the effect of the composition of the present invention is expected to be greater than the addition of the effects of each of the components of said treatment used alone, that is, of each of ZD6126 and ZD 1839 and ionising radiation, used alone.

According to another aspect of the present invention the effect of the composition of the present invention is expected to be a synergistic effect.

It should also be appreciated that according to the present invention the combination is defined as affording a synergistic effect if the effect is therapeutically superior, as measured by, for example, the extent of the response, the response rate, the time to disease progression or the survival period, to that achievable on dosing one or other of the components of the combination treatment at its conventional dose. For example, the effect of the combination is synergistic if the effect is therapeutically superior to the effect achievable with ZD6126 or ZD 1839 or ionising radiation alone. Further, the effect of the combination is synergistic if a beneficial effect is obtained in a group of patients that does not respond (or responds poorly) to ZD6126 or ZD1839 or ionising radiation alone. In addition, the effect of the combination is defined as affording a synergistic effect if one of the components is dosed at its conventional dose and the other component(s) is/are dosed at a reduced dose and the therapeutic effect, as measured by, for example, the extent of the response, the response rate, the time to disease progression or the survival period, is equivalent to that achievable on dosing conventional amounts of the components of the combination treatment. In particular, synergy is deemed to be present if the conventional dose of ZD6126 or ZD1839 or ionising radiation may be reduced without detriment to one or more of the extent of the response, the response rate, the time to disease progression and survival data, in particular without detriment to the duration of the response, but with fewer and/or less troublesome side-effects than those that occur when conventional doses of each component are used.

As stated above the combinations of the present invention as defined herein are of interest for their vascular damaging effects resulting from inappropriate angiogenesis. Such combinations of the invention are expected to be useful in the prophylaxis and treatment of a wide range of disease states where inappropriate angiogenesis occurs including cancer, (including leukaemia, multiple myeloma and lymphoma), diabetes, psoriasis, rheumatoid arthritis, Kaposi's sarcoma, haemangioma, acute and chronic nephropathies, atheroma, arterial restenosis, autoimmune diseases, acute inflammation, endometriosis, dysfunctional uterine bleeding and ocular diseases with retinal vessel proliferation including age-related macular degeneration. In particular such combinations of the invention are expected to slow advantageously the growth of primary and recurrent solid tumours of, for example, the colon, breast, prostate, lungs and skin.

The compositions described herein may be in a form suitable for oral administration, for example as a tablet or capsule, for parenteral injection (including intravenous, subcutaneous, intramuscular, intravascular or infusion) for example as a sterile solution, suspension or emulsion, for topical administration for example as an ointment or cream, for rectal administration for example as a suppository or the route of administration may be by direct injection into the tumour or by regional delivery or by local delivery. In other embodiments of the present invention the ZD6126 of the combination may be delivered endoscopically, intratracheally, intralesionally, percutaneously, intravenously, subcutaneously, intraperitoneally or intratumourally. Preferably ZD6126 is administered intravenously. In general the compositions described herein may be prepared in a conventional manner using conventional excipients. The compositions of the present invention are advantageously presented in unit dosage form.

ZD6126 will normally be administered to a warm-blooded animal at a unit dose within the range 10-500mg per square metre body area of the animal, for example approximately 0.3-15mg/kg in a human. A unit dose in the range, for example, 0.3-15mg/kg, preferably 0.5-5mg/kg is envisaged and this is normally a therapeutically-effective dose. A unit dosage form such as a tablet or capsule will usually contain, for example 25-250mg of active ingredient. Preferably a daily dose in the range of 0.5-5mg/kg is employed.

It has been reported, in International Patent Application Publication No. WO 01/74369, that the effect of a given dose of ZD6126 can be increased by administering it in divided doses. Divided doses, also called split doses, means that the total dose to be administered to a warm-blooded animal, such as a human, in any one day period (for example one 24 hour period from midnight to midnight) is divided up into two or more fractions of the total dose and these fractions are administered with a time period between each fraction of about greater than 0 hours to about 10 hours, preferably about 1 hour to about 6 hours, more preferably about 2 hours to about 4 hours. The fractions of total dose may be about equal or unequal.

For example the total dose may be divided into two parts which may be about equal with a time interval between doses of greater than or equal to two hours and less than or equal to 4 hours.

ZD6126 may be administered in divided doses when used in combination with ZD1839.

For ZD 1839, a conventional tablet formulation may be used for oral administration to humans containing 50 mg, 100 mg, 250 mg or 500 mg of active ingredient. Conveniently the daily oral dose of ZD1839 is, for example, in the range 25 to 750 mg, preferably in the range 50 to 600 mg, more preferably in the range 100 to 400mg.

Radiotherapy may be administered according to the known practices in clinical radiotherapy. The dosages of ionising radiation will be those known for use in clinical radiotherapy. The radiation therapy used will include for example the use of γ-rays, X-rays, and/or the directed delivery of radiation from radioisotopes. Other forms of DNA damaging factors are also included in the present invention such as microwaves and UV-irradiation. For example X-rays may be dosed in daily doses of 1.8-2.0Gy, 5 days a week for 5-6 weeks. Normally a total fractionated dose will lie in the range 45-60Gy. Single larger doses, for example 5-10Gy may be administered as part of a course of radiotherapy. Single doses may be administered intraoperatively. Hyperfractionated radiotherapy may be used whereby small doses of X-rays are administered regularly over a period of time, for example 0.1 Gy per hour over a number of days. Dosage ranges for radioisotopes vary widely, and depend on the half-life of the isotope, the strength and type of radiation emitted, and on the uptake by cells.

As stated above the size of the dose of each therapy which is required for the therapeutic or prophylactic treatment of a particular disease state will necessarily be varied depending on the host treated, the route of administration and the severity of the illness being treated. Accordingly the optimum dosage may be determined by the practitioner who is treating any particular patient. For example, it may be necessary or desirable to reduce the above-mentioned doses of the components of the combination in order to reduce toxicity.

The present invention relates to combinations of ZD1839 or a salt thereof with ZD6126 or a salt thereof. Salts of ZD6126 for use in pharmaceutical compositions will be pharmaceutically acceptable salts, but other salts may be useful in the production of ZD6126 and its pharmaceutically acceptable salts. Such salts may be formed with an inorganic or organic base which affords a pharmaceutically acceptable cation. Such salts with inorganic or organic bases include for example an alkali metal salt, such as a sodium or potassium salt, an alkaline earth metal salt such as a calcium or magnesium salt, an ammonium salt or for example a salt with methylamine, dimethylamine, trimethylamine, piperidine, morpholine or tris-(2-hydroxyethyl)amine.

Salts of ZD 1839 for use in pharmaceutical compositions will be pharmaceutically acceptable salts, but other salts may be useful in the production of ZD1839 and its pharmaceutically acceptable salts. Salts include, for example, an acid-addition salt of ZD1839, for example, a mono- or di-acid-addition salt with, for example, an inorganic or organic acid, for example hydrochloric, hydrobromic, sulphuric, phosphoric, trifluoroacetic, citric, maleic, tartaric, fumaric, methanesulphonic or 4-toluenesulphonic acid.

ZD6126 may be made according to the following process.

N-Acetylcolchinol (30.0g, 83.9mmol) is dissolved in acetonitrile under an inert atmosphere and 1,2,3-triazole (14.67g, 212.4mmol) added via a syringe. Di-tert-butyl-diethylphosphoramidite (37.7g, 151.4mmol) is added and the reaction mixture stirred at about 20°C to complete the formation of the intermediate phosphite ester. Cumene hydroperoxide (24.4g, 159.2mmol) is added at about 10°C and the reaction mixture stirred until the oxidation is complete. Butyl acetate (50ml) and sodium hydroxide solution (250ml of 1M) are added, the reaction mixture stirred and the aqueous phase discarded. The organic solution is washed with sodium hydroxide solution (2 x 250ml of 1M) and a saturated solution of sodium chloride. Trifluoroacetic acid (95.3g, 836mmol) is added at about 15°C. The reaction mixture is distilled at atmospheric pressure, ZD6126 crystallises and is isolated at ambient temperature.

ZD1839 may be synthesised according to any of the known processes for making ZD1839. For example ZD1839 may be made according to the processes described in WO 96/33980 (See Examples 1, 10 and 24-31).

The following test may be used to demonstrate the activity of ZD6126 in combination with ZD1839.

### LoVo Tumour Model

Athymic nude mice were implanted subcutaneously with 1.5 x 10⁷ human LoVo tumour cells (obtained from European Collection of Cell Cultures, ECACC, CAMR, Salisbury, Wiltshire, SP4 0JG, UK ; Cat. no. CCL 229) and tumours allowed to grow until they were approximately 9mm in diameter. Groups of 8 tumour bearing mice were then treated as follows:
Control animals were dosed with PBSA 0.1ml/10g intraperitoneally (i.p.) single bolus on day 0 then about 2h hours later 0.1% Tween 80 0.1ml/10g orally (p.o.) on day 0, followed by 0.1% Tween 80 0.1ml/10g p.o. once daily on days 1-13;
ZD6126 alone was dosed 200 mg/kg i.p. on day 0;
ZD1839 alone was dosed 200 mg/kg/day p.o. on days 0-13; and
(in the combination arm) ZD6126 200mg/kg i.p. on day 0 then about 2 hours later ZD1839 200mg/kg p.o. dosed on day 0, followed by ZD1839 200mg/kg p.o. once daily on days 1-13. Tumour growth was measured twice weekly from calliper measurements of tumour diameter and the time taken for tumours to double in size compared using the Mann-Whitney U statistic.

### Results

| Group | Growth delay in days for tumours to double in size |
|---|---|
| ZD6126 only | 3.6 days; p<0.05 |
| ZD1839 only | 8.3 days; p<0.01 |
| ZD6126 + ZD1839 | 14.0 days; p<0.01 |

The tumour growth delay caused by the combination of ZD6126 and ZD1839 was significantly (Mann-Whitney U-test) greater than either ZD1839 alone (P<0.01) or ZD6126 alone (P<0.05). The growth delay from the combination was greater than the sum of the growth delays from the individual treatments.

The following test may be used to demonstrate the activity of ZD6126 in combination with ZD1839 and optionally with ionising radiation.

### A549 NSCLC xenograft model

A human non-small cell lung cancer (NSCLC) xenograft model was used. Athymic nude mice were injected subcutaneously (s.c.) with A549 human NSCLC cells. Treatment began after 7 days when tumours were established (tumour volume = 100-300 mm³). Groups of animals (N = 5-10 per group) received single agent treatment with 4 Gy of radiotherapy (RT) for 2 consecutive days each week for 2 weeks (days 8, 9, 15, 16), or treatment with ZD6126 (150 mg/kg intraperitoneally (i.p)) for 1 day each week for 2 weeks (days 10, 17), or treatment with ZD1839 (2.5 mg per day orally (p.o.)) from days 7-11 and days 14-18. Additional groups of animals received 2-agent or 3-agent combinations of RT, ZD6126 and ZD1339, using the same doses and schedules used for single agent treatment (N = 5 - 10 per group). Animals in all groups were sacrificed on day 42 after tumour cell implantation, when the control tumours reached approximately 2.0 cm³.

### Results

Compared with control animals, RT, ZD6126 and ZD1839 induced a marked inhibition of tumour growth. The combination of ZD6126/ZD1839 or ZD6126/RT or ZD1839/RT resulted in greater inhibition than any single agent alone. The triple combination of ZD6126, ZD1839 and RT induced the greatest effects on tumour growth.

| Treatment Group | Tumour size¹ (cm³ ± S.D.) |
|---|---|
| Control | 1.83 ± 0.10 |
| RT | 0.90 ± 0.10 |
| ZD6126 | 1.17 ± 0.10 |
| ZD1839 | 1.41 ± 0.08 |
| RT + ZD6126 | 0.35 ± 0.06 |
| RT + ZD1839 | 0.26 ± 0.04 |
| ZD6126 + ZD1839 | 0.43 ± 0.07 |
| RT+ZD6126 + ZD1839 | 0.15 ± 0.02 |

| | |
|---|---|
| ¹Measurements on day 42 after tumour implantation The results are presented graphically in Figure 1. | |

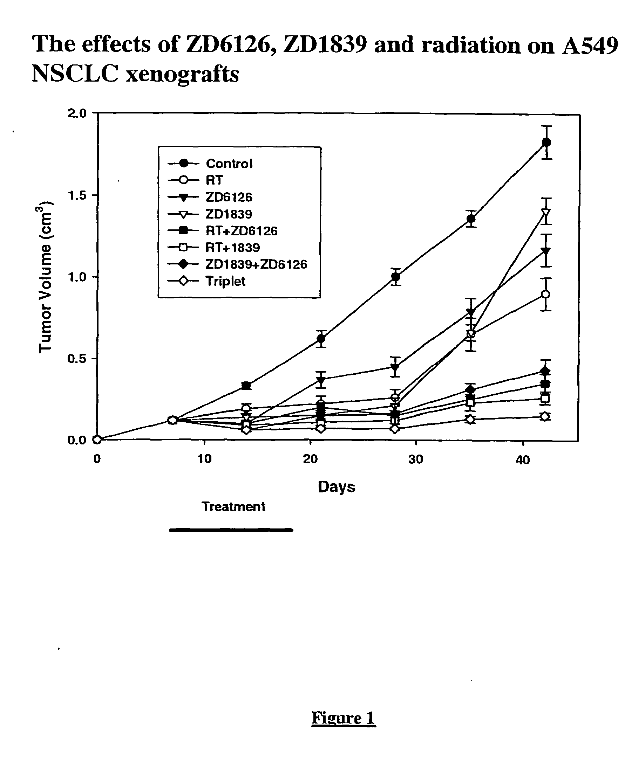

## Claims

1. A pharmaceutical composition which comprises ZD6126 or a pharmaceutically acceptable salt thereof, and ZD1839 or a pharmaceutically acceptable salt thereof, in association with a pharmaceutically acceptable excipient or carrier.

2. A kit comprising ZD6126 or a pharmaceutically acceptable salt thereof, and ZD1839 or a pharmaceutically acceptable salt thereof.

3. Use of ZD6126 or a pharmaceutically acceptable salt thereof and ZD1839 or a pharmaceutically acceptable salt thereof in the manufacture of a medicament for use in the production of a vascular damaging effect resulting from inappropriate angiogenesis in a warm-blooded animal such as a human.

4. Use of ZD6126 or a pharmaceutically acceptable salt thereof and ZD 1839 or a pharmaceutically acceptable salt thereof in the manufacture of a medicament for use in the production of a vascular damaging effect resulting from inappropriate angiogenesis in a warm-blooded animal such as a human which is being treated with ionising radiation.

5. Use of ZD6126 or a pharmaceutically acceptable salt thereof and ZD1839 or a pharmaceutically acceptable salt thereof in the manufacture of a medicament for use in the production of an anti-cancer effect in a warm-blooded animal such as a human.

6. Use of ZD6126 or a pharmaceutically acceptable salt thereof and ZD1839 or a pharmaceutically acceptable salt thereof in the manufacture of a medicament for use in the production of an anti-cancer effect in a warm-blooded animal such as a human which is being treated with ionising radiation.

7. Use of ZD6126 or a pharmaceutically acceptable salt thereof and ZD1839 or a pharmaceutically acceptable salt thereof in the manufacture of a medicament for use in the production of an anti-tumour effect in a warm-blooded animal such as a human.

8. Use of ZD6126 or a pharmaceutically acceptable salt thereof and ZD1839 or a pharmaceutically acceptable salt thereof in the manufacture of a medicament for use in the production of an anti-tumour effect in a warm-blooded animal such as a human which is being treated with ionising radiation.

9. Use of ZD6126 or a pharmaceutically acceptable salt thereof and ZD1839 or a pharmaceutically acceptable salt thereof in the manufacture of a medicament for use in the treatment of a cancer involving a solid tumour in a warm-blooded animal such as a human.

10. Use of ZD6126 or a pharmaceutically acceptable salt thereof and ZD1839 or a pharmaceutically acceptable salt thereof in the manufacture of a medicament for use in the treatment of a cancer involving a solid tumour in a warm-blooded animal such as a human which is being treated with ionising radiation.

11. The use according to any one of claims 3 to 10 wherein the total dose of the ZD6126 or a pharmaceutically acceptable salt thereof, to be administered to the warm-blooded animal in any one day period, is divided into two or more fractions of the total dose and these fractions are administered to the warm-blooded animal with a time period between each fraction of greater than 0 hours to about 10 hours.

## Patentansprüche

1. Pharmazeutische Zusammensetzung, enthaltend ZD6126 oder ein pharmazeutisch annehmbares Salz davon und ZD1839 oder ein pharmazeutisch annehmbares Salz davon zusammen mit einem pharmazeutisch annehmbaren Hilfsstoff oder Träger.

2. Kit, enthaltend ZD6126 oder ein pharmazeutisch annehmbares Salz davon und ZD1839 oder ein pharmazeutisch annehmbares Salz davon.

3. Verwendung von ZD6126 oder einem pharmazeutisch annehmbaren Salz davon und ZD1839 oder einem pharmazeutisch annehmbaren Salz davon bei der Herstellung eines Arzneimittels zur Verwendung bei der Erzielung einer aus unangemessener Angiogenese resultierenden gefäßschädigenden Wirkung bei einem Warmblüter wie dem Menschen.

4. Verwendung von ZD6126 oder einem pharmazeutisch annehmbaren Salz davon und ZD1839 oder einem pharmazeutisch annehmbaren Salz davon bei der Herstellung eines Arzneimittels zur Verwendung bei der Erzielung einer aus unangemessener Angiogenese resultierenden gefäßschädigenden Wirkung bei einem Warmblüter wie dem Menschen, der mit ionisierender Strahlung behandelt wird.

5. Verwendung von ZD6126 oder einem pharmazeutisch annehmbaren Salz davon und ZD1839 oder einem pharmazeutisch annehmbaren Salz davon bei der Herstellung eines Arzneimittels zur Verwendung bei der Erzielung einer Antikrebswirkung bei einem Warmblüter wie dem Menschen.

6. Verwendung von ZD6126 oder einem pharmazeutisch annehmbaren Salz davon und ZD1839 oder einem pharmazeutisch annehmbaren Salz davon bei der Herstellung eines Arzneimittels zur Verwendung bei der Erzielung einer Antikrebswirkung bei einem Warmblüter wie dem Menschen, der mit ionisierender Strahlung behandelt wird.

7. Verwendung von ZD6126 oder einem pharmazeutisch annehmbaren Salz davon und ZD1839 oder einem pharmazeutisch annehmbaren Salz davon bei der Herstellung eines Arzneimittels zur Verwendung bei der Erzielung einer Antitumorwirkung bei einem Warmblüter wie dem Menschen.

8. Verwendung von ZD6126 oder einem pharmazeutisch annehmbaren Salz davon und ZD1839 oder einem pharmazeutisch annehmbaren Salz davon bei der Herstellung eines Arzneimittels zur Verwendung bei der Erzielung einer Antitumorwirkung bei einem Warmblüter wie dem Menschen, der mit ionisierender Strahlung behandelt wird.

9. Verwendung von ZD6126 oder einem pharmazeutisch annehmbaren Salz davon und ZD1839 oder einem pharmazeutisch annehmbaren Salz davon bei der Herstellung eines Arzneimittels zur Verwendung bei der Behandlung einer Krebserkrankung mit einem soliden Tumor bei einem Warmblüter wie dem Menschen.

10. Verwendung von ZD6126 oder einem pharmazeutisch annehmbaren Salz davon und ZD1839 oder einem pharmazeutisch annehmbaren Salz davon bei der Herstellung eines Arzneimittels zur Verwendung bei der Behandlung einer Krebserkrankung mit einem soliden Tumor bei einem Warmblüter wie dem Menschen, der mit ionisierender Strahlung behandelt wird.

11. Verwendung nach einem der Ansprüche 3 bis 10, bei der die Gesamtdosis des dem Warmblüter über einen Zeitraum von einem Tag zu verabreichenden ZD6126 oder eines pharmazeutisch annehmbaren Salzes davon in zwei oder mehr Fraktionen der Gesamtdosis geteilt wird und diese Fraktionen dem Warmblüter so verabreicht werden, daß der Zeitraum dazwischen mehr als 0 Stunden bis etwa 10 Stunden beträgt.

## Revendications

1. Composition pharmaceutique comprenant du ZD6126 ou un sel pharmaceutiquement acceptable de celui-ci, et du ZD1839 ou un sel pharmaceutiquement acceptable de celui-ci, en association avec un excipient ou un support pharmaceutiquement acceptable.

2. Kit comprenant du ZD6126 ou un sel pharmaceutiquement acceptable de celui-ci, et du ZD1839 ou un sel pharmaceutiquement acceptable de celui-ci.

3. Utilisation du ZD6126 ou d'un sel pharmaceutiquement acceptable de celui-ci, et du ZD1839 ou d'un sel pharmaceutiquement acceptable de celui-ci, pour la fabrication d'un médicament destiné à être utilisé pour la production d'un effet de détérioration vasculaire résultant d'une angiogénèse inappropriée chez un animal à sang chaud, tel qu'un être humain.

4. Utilisation du ZD6126 ou d'un sel pharmaceutiquement acceptable de celui-ci, et du ZD1839 ou d'un sel pharmaceutiquement acceptable de celui-ci, pour la fabrication d'un médicament destiné à être utilisé pour la production d'un effet de détérioration vasculaire résultant d'une angiogénèse inappropriée chez un animal à sang chaud, tel qu'un être humain qui est traité par un rayonnement ionisant.

5. Utilisation du ZD6126 ou d'un sel pharmaceutiquement acceptable de celui-ci, et du ZD1839 ou d'un sel pharmaceutiquement acceptable de celui-ci, pour la fabrication d'un médicament destiné à être utilisé pour la production d'un effet anti-cancéreux chez un animal à sang chaud, tel qu'un être humain.

6. Utilisation du ZD6126 ou d'un sel pharmaceutiquement acceptable de celui-ci, et du ZD1839 ou d'un sel pharmaceutiquement acceptable de celui-ci, pour la fabrication d'un médicament destiné à être utilisé pour la production d'un effet anti-cancéreux chez un animal à sang chaud, tel qu'un être humain qui est traité par un rayonnement ionisant.

7. Utilisation du ZD6126 ou d'un sel pharmaceutiquement acceptable de celui-ci, et du ZD1839 ou d'un sel pharmaceutiquement acceptable de celui-ci, pour la fabrication d'un médicament destiné à être utilisé pour la production d'un effet anti-tumoral chez un animal à sang chaud, tel qu'un être humain.

8. Utilisation du ZD6126 ou d'un sel pharmaceutiquement acceptable de celui-ci, et du ZD1839 ou d'un sel pharmaceutiquement acceptable de celui-ci, pour la fabrication d'un médicament destiné à être utilisé pour la production d'un effet anti-tumoral chez un animal à sang chaud, tel qu'un être humain qui est traité par un rayonnement ionisant.

9. Utilisation du ZD6126 ou d'un sel pharmaceutiquement acceptable de celui-ci, et du ZD1839 ou d'un sel pharmaceutiquement acceptable de celui-ci, pour la fabrication d'un médicament destiné à être utilisé pour le traitement d'un cancer impliquant une tumeur solide chez un animal à sang chaud, tel qu'un être humain.

10. Utilisation du ZD6126 ou d'un sel pharmaceutiquement acceptable de celui-ci, et du ZD1839 ou d'un sel pharmaceutiquement acceptable de celui-ci, pour la fabrication d'un médicament destiné à être utilisé pour le traitement d'un cancer impliquant une tumeur solide chez un animal à sang chaud, tel qu'un être humain qui est traité par un rayonnement ionisant.

11. Utilisation selon l'une quelconque des revendications 3 à 10, dans laquelle la dose totale du ZD6126 ou d'un sel pharmaceutiquement acceptable de celui-ci, destinée à être administrée à l'animal à sang chaud en une période quelconque d'un jour, est divisée en deux fractions ou plus de la dose totale et ces fractions sont administrées à l'animal à sang chaud avec une période de temps entre chaque fraction de plus de 0 heure à environ 10 heures.
